# EUROPÄISCHE PATENTSCHRIFT

(11) **EP 0 741 740 B1**
(45) Veröffentlichungstag und Bekanntmachung des Hinweises auf die Patenterteilung: **07.04.1999**
(21) Anmeldenummer: 95905641.7
(22) Anmeldetag: 21.01.1995
(51) Int. Cl.: C07H 19/02

(54) **VERFAHREN ZUR HERSTELLUNG VON UNSYMMETRISCHEN PHOSPHORSÄUREDIESTERN UND DEREN SALZE**
METHOD OF PREPARING UNSYMMETRICAL PHOSPHORIC ACID DIESTERS AND SALTS
PROCEDE DE FABRICATION DE DIESTERS D'ACIDE PHOSPHORIQUE ASYMETRIQUES ET DE LEURS SELS

(30) Priorität: 28.01.1994 DE 4402492
(43) Veröffentlichungstag der Anmeldung: 13.11.1996
(73) Patentinhaber: Roche Diagnostics GmbH, 68298 Mannheim (DE)
(72) Erfinder: KIEGEL, Einhard, D-68165 Mannheim (DE); ZILCH, Harald, D-68305 Mannheim (DE)
(86) Internationale Anmeldenummer: EP9500219
(87) Internationale Veröffentlichungsnummer: WO9520596

(56) Entgegenhaltungen:
- EP-A- 0 575 717
- WO-A-91/12256
- WO-A-91/15494
- DD-A- 138 213
- J. BIOL. CHEM., Bd. 266, 1991 Seiten 11714-17, K. Y. HOSTETLER ET AL. 'Phosphatidylazidothymidine' in der Anmeldung erwähnt
- DATABASE BEILSTEIN Beilstein Informationssysteme BRN=3756203,

## Beschreibung

Gegenstand der vorliegenden Erfindung ist ein Verfahren zur Herstellung von Phosphorsäurediestern mit zwei nicht identischen organischen Resten.

Das erfindungsgemäße Verfahren zur Herstellung von Phosphorsäurediestern ist dadurch gekennzeichnet, daß man
[a] einen Phosphorsäureester der Formel I,

   R¹-O-P(O)(OH)₂ (I),

   in dem R¹ einen Lipidrest der allgemeinen Formel IV bedeutet 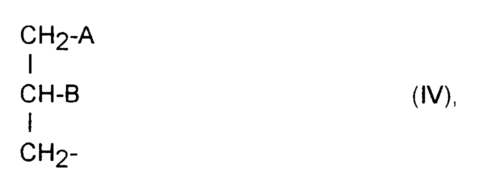
   in der A und B gleich oder verschieden sein können und Wasserstoff, C₁-C₁₈-Alkyl, C₁-C₁₈-Alkoxy, C₁-C₁₈-Alkylthio, C₁-C₁₈-Alkylsulfinyl oder C₁-C₁₈-Alkylsulfonyl bedeuten,
   oder in dem R¹ eine geradkettige oder verzweigte, gesättigte oder ungesättigte Alkylkette mit 10-20 Kohlenstoffatomen bedeutet, die gegebenenfalls ein- oder mehrfach substituiert sein kann durch Halogen, C₁-C₆-Alkoxy, C₁-C₆-Alkylmercapto mit einem Alkohol der Formel II

      R² - OH (II)
   in dem R² einen organischen Rest darstellt,
   in Gegenwart eines Arylsulfonsäurechlorids, eines sterisch gehinderten Carbonsäurechlorids oder Phosphorsäurechlorids und einer organischen Base kondensiert,
[b] den entstandenen Phosphorsäurediester durch Zugabe einer Erdalkali-lonen-haltigen Lösung als schwerlösliches Salz fällt und isoliert,
[c] das schwerlösliche Salz durch Suspendieren in einem mit Wasser nicht mischbaren organischen Lösemittel und einer verdünnten wäßrigen Mineralsäure als freie Säure im organischen Lösemittel isoliert .

Anschließend wird das nach Schritt [c] erhaltene Rohprodukt durch präparative Chromatographie an einer entsprechenden Säule, beispielsweise einer Umkehrphasen-Säule (RP=Reversed Phase) gereinigt (Schritt [d]) und anschließend die in Form der freien Säure erhaltene Verbindung in ein beliebiges physiologisch verträgliches Salz überführt Schritt [e]).

In bestimmten Fällen, in denen der Alkohol der Formel II z. B. neben einer primären OH-Gruppe noch weitere sekundäre OH-Gruppen besitzt, kann es von Vorteil sein, den Phosphorsäureester der Formel I mit dem Arylsulfonsäurechlorid in Gegenwart der organischen Base zur Reaktion zu bringen (Erzeugung des gemischten Anhydrids) und erst anschließend den Alkohol der Formel II zuzugeben.

Das beanspruchte Verfahren ist besonders geeignet zur Herstellung von Liponucleotiden, wobei R² einen nucleosidischen Rest darstellt.

In WO 92/03462 sind drei verschiedene Verfahren zur Herstellung von Lipidderivaten von Nucleosiden aufgezeigt:
1. Umsetzung des Lipidphosphatdichlorids mit dem Nucleosid in Gegenwart einer organischen Base.
2. Enzymatische Katalyse der Reaktion des Lipidphosphorsäuremonocholinesters mit dem Nucleosid (Phospholipase D aus Streptomyces).
3. Kondensation des Lipidphosphats mit dem Nucleosid in Gegenwart von DCC (Dicyclohexylcarbodiimid).

Alle drei Varianten sind sowohl zur Knüpfung internucleotidischer Bindungen, als auch zur Kondensation von Nucleosiden mit Lipidphosphaten geeignet und in der Literatur beschrieben.

In J. Med. Chem. 34, 1408 (1991), Biochem. Biophys. Res. Commun. 171, 451 (1990) und US 5,149,794 ist die Kondensation eines Lipidphosphats und eines Nucleosids mit DCC beschrieben. In Biochem. 31, 4757 (1992), EP 0 457 570, EP 0 262 876 und WO 92/17487 ist die enzymatisch katalysierte Kondensation eines Phosphocholins mit einem Nucleosid in Gegenwart von Phospholipase D aus Streptomyces beschrieben. Darüber hinaus sind von Khorana et.al. in verschiedenen Publikationen [z.B. J. Am. Chem. Soc. 88, 829 (1966), dto. 86, 1630 (1964)] Arylsulfonsäurechloride für die Synthese internucleotidischer Bindungen propagiert worden, insbesondere das sterisch gehinderte 2,4,6-Triisopropylbenzolsulfonsäurechlorid. Für die Kondensation von Phospholipiden mit Nucleosiden wurde dieses Reagenz von Hostetler et. al. eingesetzt, beschrieben u.a. in J. Biol. Chem. 265, 6112 (1990), dto. 266, 11714 (1991). Analog gelang die Kondensation von Nucleosid-Monophosphaten mit primären Lipidalkoholen, wie in J. Biol. Chem. 267, 20288 (1992).

In EP-A-0575717 und WO-A-9115494 werden Ca-Salze von Glycerylphosphorylserinen beschrieben. Diese Ca-Salze von Carbonsäuren sind gut löslich. In WO-91-12256 ist das gut lösliche Ca-Salz eines unsymmetrischen Phosphorsäurediesters beschrieben. Die Patentschrift DD-A-138213 beschreibt die Überführung von Phosphorsäurediestern in ein Ba-Salz.

Die beschriebenen Methoden haben jedoch den Nachteil. daß die Herstellung von Lipidderivaten nur in unbefriedigenden Ausbeuten möglich ist. Weitere Nachteile sind außerdem mangelnde Produktreinheiten und aufwendige Reinigungsverfahren. die die Technisierung im großtechnischen Maßstab von beispielsweise mehreren Kilogramm-Mengen erschweren. Selbst die Reproduktion der publizierten Verfahren gelang nicht in den beschriebenen Ausbeuten bei größeren Verfahrensansätzen.

Mit dem erfindungsgemäßen Verfahren konnten diese Schwierigkeiten beseitigt werden. Eine weitere Aufgabe der Erfindung war, ein Verfahren zur Verfügung zu stellen, mit dem grenzflächenaktive Phosphorsäurediester gereinigt werden können. Das Endprodukt des Verfahrens erlaubt außerdem direktes Vermahlen.

Insbesondere ist Gegenstand der vorliegenden Erfindung ein Verfahren zur Herstellung von Lipidderivaten, das dadurch gekennzeichnet ist, daß man
[a] einen Phosphorsäureester der Formel I, in dem R¹ einen speziellen Lipidteil darstellt, mit einem 5'-ungeschützten Nucleosid in Gegenwart eines Arylsulfonsäurechlorids und einer organischen Base wie z. B. Pyridin kondensiert,
[b] nach der Hydrolyse den Eindampfrückstand mit einem organischen Lösemittel (z.B. Dipe, MTB) ausrührt, das entstandene Arylsulfonsäure-Pyridinsalz nahezu vollständig zur Kristallisation bringt und recycliert,
[c] das Liponucleotid durch Zugabe von z.B. wäßriger Calciumacetat-Lösung als schwerlösliches Salz fällt und isoliert,
[d] das schwerlösliche Salz durch Suspendieren in einem mit Wasser nicht mischbaren organischen Lösungsmittel und einer verdünnten wäßrigen Mineralsäure als freie Säure im organischen Lösemittel isoliert (Eindampfrückstand >95% Fläche nach HPLC).

Das Rohprodukt wird anschließend durch präparative Chromatographie an einer RP-Phase gereinigt und die freie Säure in ein geeignetes Salz überführt.

Nach der HPLC ist gegebenenfalls eine Zwischenisolierung aus den produktenthaltenden Fraktionen durch Fällung des Calciumsalzes möglich, mit anschließender Überführung in die freie Säure und ins Natriumsalz (wie oben beschrieben).

Nur in dieser besonderen Kombination verschiedener Verfahrensschritte zeigt sich ein entscheidender Fortschritt, der eine Produktion entsprechender Verbindungen im Multi-kg-Maßstab in ökonomischer Weise erlaubt.

Die Kondensation in Gegenwart von Arylsulfonsäurechlorid gelingt ebenfalls bei Verwendung des Nucleosid-Monophosphats und des Lipidalkohols, wobei die anschließenden Verfahrensschritte identisch sind.

Das verwendete Lipidphosphat kann als Rohprodukt eingesetzt werden, ohne einen signifikanten Einfluß auf die Produktreinheit oder die Ausbeute bezogen auf Nucleosid. Als Base eignen sich z.B. Pyridin oder Lutidin in einem inerten organischen Lösungsmittel, wie z.B. Toluol, oder die Umsetzung wird direkt in der Base ohne weiteres Lösungsmittel durchgeführt.

Als Arylsulfonsäurechloride kommen Benzol-, Toluol-, 2,4,6-Trimethylbenzol-, 2,6-Dimethylbenzol-, 2,4,6-Triisopropylbenzol- oder 2,6-Diisopropylbenzolsulfonsäurechlorid in Frage. Je sperriger die Substituenten in den ortho-Positionen sind, desto weniger Nebenprodukte sind zu erwarten.

Anstelle des Arylsulfonsäurechlorids kann in einigen Fällen auch ein sterisch gehindertes Carbonsäurechlorid oder Phosphorsäurechlorid eingesetzt werden.

Beispielhaft ist als verwendbares Carbonsäurechlorid das Pivaloylchlorid zu nennen. Als aktive Zwischenstufe für die Kondensation mit dem Nucleosid ist unter Verwendung eines Phosphorsäurechlorids auch eine Verbindung der Formel III zu verstehen, die formal ein Anhydrid des Phosphats der Formel I darstellt.

R¹-O-P(O)(OH)-O-P-(O)(OH)-O-R¹ (III)

Für die Fällung des schwerlöslichen Salzes wird bevorzugt Calcium in Form seines Acetats, Hydroxids, Carbonats oder Hydrids verwendet, aufgrund seiner guten physiologischen Verträglichkeit und der schlechten Löslichkeit des Salzes im organischen Lösungsmittel.

Die chromatographische Reinigung wird bevorzugt an einer Umkehrphase mit Methanol/Pufferlösung als Eluens durchgeführt. Auf der Endstufe wird die isolierte freie Säure in ein physiologisch verträgliches Salz, z.B. ein Kalium-, Lithium- oder Natriumsalz, überführt.

Im Sinne der vorliegenden Erfindung ist R¹ ein Lipidrest der allgemeinen Formel IV 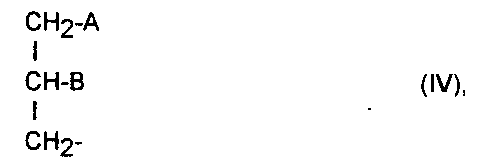 in der A und B gleich oder verschieden sein können und Wasserstoff, C₁-C₁₈-Alkyl, C₁-C₁₈-Alkoxy, C₁-C₁₈-Alkylthio, C₁-C₁₈-Alkylsulfinyl oder C₁-C₁₈-Alkylsulfonyl bedeuten oder R¹ ist eine geradkettige oder verzweigte, gesättigte oder ungesättigte Alkylkette mit 10-20 Kohlenstoffatomen, die gegebenenfalls ein- oder mehrfach substituiert sein kann durch Halogen C₁-C₆-Alkoxy, C₁-C₆-Alkylmercapto.
- R²: stellt bevorzugt einen 5'-Nucleosidrest der allgemeinen Formel V dar 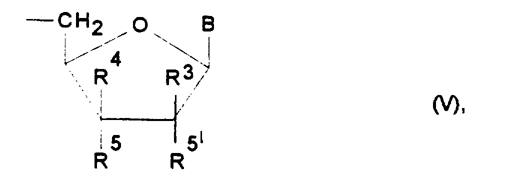
in der
- R³: Wasserstoff, Halogen oder eine Hydroxygruppe,
- R⁴, R⁵: jeweils Wasserstoff oder einer der Reste R⁴ und R⁵ Halogen, eine Hydroxy-, eine Cyano-, eine Amino- oder eine Azidogruppe bedeuten und außerdem R³ und R⁴ eine weitere Bindung zwischen C-2' und C-3' darstellen können,
- R^{5'}: Wasserstoff, Hydroxy, Azido, Amino, Cyano oder Halogen bedeutet,
oder einen seco-Nucleosidrest der allgemeinen Formel V a darstellt 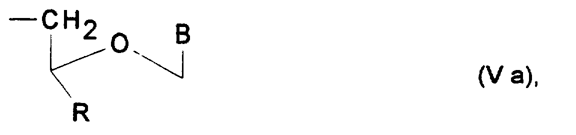
in dem
- R: ein Wasserstoffatom oder eine gegebenenfalls durch Hydroxy, Halogen oder Azido substituierte C₁-C₃-Alkylgruppe darstellt,
und
- B: eine der folgenden Verbindungen bedeutet:

1.) 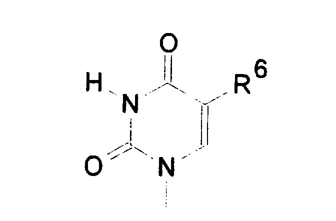 wobei
   - R⁶: Wasserstoff, eine Alkylkette mit 1 - 4 Kohlenstoffatomen, die durch Halogen substituiert sein kann, ein Alkenyl- bzw. Alkinylrest mit 2 - 6 Kohlenstoffatomen, der gegebenenfalls durch Halogen substituiert sein kann, oder Halogen sein kann,
2.) 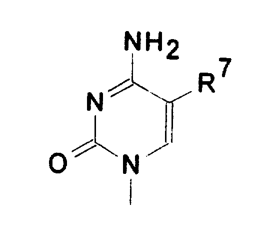 wobei
   - R⁷: Wasserstoff, eine Alkylkette mit 1 - 4 Kohlenstoffatomen, die durch Halogen substituiert sein kann, oder Halogen sein kann,
3.) 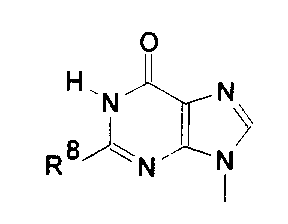 wobei
   - R⁸: Wasserstoff, eine Alkylkette mit 1 - 4 Kohlenstoffatomen. Halogen, oder eine Hydroxy- oder eine Aminogruppe sein kann,
4.) 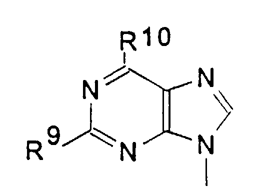 wobei
   - R⁹: Wasserstoff, Halogen oder eine Aminogruppe sein kann, und
   - R¹⁰: Wasserstoff, Halogen, Mercapto, C₁-C₆-Alkoxy, C₁-C₆-Alkylmercapto, oder eine Aminogruppe, die mono- oder disubstituiert sein kann durch C₁-C₆-Alkyl-, C₁-C₆-Alkoxy-, Hydroxy-C₂-C₆-alkyl- und/oder C₃-C₆-Cycloalkyl-, Aryl-, Hetaryl-, Aralkyl- oder Hetarylalkylgruppen, die gegebenenfalls im Aryl- oder Hetarylrest noch durch eine oder mehrere Hydroxy-, Methoxy- oder Alkylgruppen oder Halogen substituiert sein können, oder Allyl, das gegebenenfalls mit Mono- oder Dialkyl- oder Alkoxygruppen substituiert sein kann, bedeutet.

Bevorzugt bedeutet R² z. B. einen Rest ausgewählt aus der Gruppe:
-2',3'Didesoxy-3'-azidouridin
-2',3'-Didesoxyinosin
-2',3'-Didesoxyguanosin
-2',3'-Didesoxycytidin
-2',3'-Didesoxyadenosin
-3'-Desoxythymidin
-2',3'-Didesoxy-2',3'-didehydro-N⁶-(o-methylbenzyl)-adenosin
-2',3'-Didesoxy-2',3'-didehydro-N⁶-(2-methylpropyl )-adenosin
-2',3'-Didesoxy-3'-azidoguanosin
-3'-Desoxy-3'-azido-thymidin
-2',3'-Didesoxy-3'-fluor-5-chloruridin
-3'-Desoxy-3'-fluorthymidin
-2',3'-Didesoxy-3'-fluoradenosin
-2',3'-Didesoxy-3'-fluor-2,6-diaminopurinribosid
-2',3'-Didesoxy-2',3'-didehydrocytidin
-3'-Desoxy-2',3 '-didehydrothymidin
-3'-Desoxy-3'-azidothymidin
-5-Fluoruridin
-5-Trifluormethyl-2'-desoxyuridin
-6-Mercaptopurin-9-β-D-ribofuranosid
6-Methylmercaptopurin-9-β-D-ribofuranosid
-2-Fluoradenin-9-β-D-arabinofuranosid
-2-Chlor-2'-desoxyadenosin
-Acyclovir
-Ganciclovir

### Beispiel 1

### Herstellung des Rohprodukts von (3'-Desoxy-3'-azido-thymidin)-5'-phosphorsäure-(3-dodecylthio-2-decyloxy-)propylester

509 g (0.95 Mol) 3-Dodecylthio-2-decyloxy-propanol-1-monophosphat werden in 2.84 L trocknem Pyridin bei Raumtemperatur gelöst. 443.8 g (1.467 Mol) Triisopropylbenzolsulfochlorid werden der Lösung zugegeben, gefolgt von 229 g (0.857 Mol) AZT; nach ca.10 min Rühren bei Raumtemperatur ist alles gelöst. Die braune Lösung wird über Nacht bei Raumtemperatur gerührt. Man setzt dann 1.43 L Wasser zu und rührt 30 min bei Raumtemperatur nach; bei der Wasserzugabe trübt sich das Gemisch, wird aber beim Nachrühren wieder klar. Die Lösung wird bei ca. 60 °C Badtemperatur i. Vak. am Rotationsverdampfer eingeengt und der Eindampfrückstand wird zweimal mit je 2.8 L Toluol zum Entfernen von Pyridin nachdestilliert. Der halbfeste Eindampfrückstand wird mit 5.7 L Diisopropylether übergossen und ca. 1 Std. im Eisbad gerührt. Man saugt das Salz (Pyridinsalz der Triisopropylbenzolsulfonsäure) ab und wäscht mit 700 ml Diisopropylether portionsweise nach. Das Salz wird bei 50 °C getrocknet (615 g) und gestapelt bzw. recycliert. Das Etherfiltrat wird dreimal mit je 1.8 L 2n HCI ausgeschüttelt. Sollte keine gute Phasentrennung erreicht werden, wird die Emulsionsschicht vorsichtig über eine Fibrazellschicht gesaugt. Man trocknet die organische Phase mit Natriumsulfat und engt gewichtskonstant ein.

Auswaage 677 g Phosphorsäurediester roh ("106%" d. Th. bez auf AZT) HPLC: ca. 76 Fl-% (Flächenprozent).

### Beispiel 2

### Reinigung des rohen Phosphorsäurediesters durch Ca-Salzbildung

283 g (0.38 Mol) des Rohprodukts aus Beispiel 1 werden in 1.73 L Methanol und 850 ml Wasser unter Rühren bei Raumtemperatur gelöst. Eine Lösung von 60.1 g Ca-Acetat-Hydrat in 400ml Wasser wird bei Raumtemperatur in ca. 45 min unter Rühren eingetropft; es bildet sich eine zunächst schmierige Fällung, die längere Zeit (z.B.über Nacht) nachgerührt wird. Dabei wird der Niederschlag pulvrig. Das Calciumsalz wird abgesaugt und nutschenfeucht mit 1 L Aceton bei Raumtemperatur ca. 30 min intensiv ausgerührt. Man saugt ab und trocknet. Auswaage 192 g.

Das Calciumsalz wird in 1.2 L Methyl-tert.butylether und 276 ml 2n HCI suspendiert und intensiv gerührt bis das Salz zerfallen ist und sich eine leicht trübe Wasserphase gebildet hat. Nach Phasentrennung wird die MTB-Phase 2x mit je 250ml gesättigter NaCl-Lösung gewaschen, über Na₂SO₄ getrocknet und eingeengt

Eindampfrückstand: 179.1 g (63.2% der eingesetzten Menge) HPLC 90.46 Fl-%.

### Beispiel 3

### Reinigung des Rohprodukts durch präp.HPLC

Präparatives System:

| | |
|---|---|
| Stationäre Phase | Merck LiChroprep RP 18; 15-25µ |
| Mobile Phase | 0.02 molare NaH₂PO₄-Lösung 12.5% + Methanol 87.5% |

125 g des Rohprodukts aus Beispiel 2 werden bei ca. 35 °C in 750 ml Methanol gelöst. Man setzt 1.5 L mobile Phase zu und stellt mit conc. NaOH auf pH 5. Die Lösung wird filtriert und das Filtrat mit mobiler Phase auf 2.5 L aufgefüllt. Diese Lösung wird in 500 ml-Portionen chromatographiert.

Man fraktioniert vom Maximum des Hauptpeaks an und schneidet eine Fraktion von 1.2 L. Die vereinigten Reinfraktionen aus 5 Trennungen (6 L ) werden am Rotationsverdampfer bei 40 ° C Badtemperatur i. Vak. zu einem viscosen Rückstand eingeengt. Der Rückstand wird portionsweise mit insgesamt 500 ml Wasser aus der Apparatur gespült und mit halbconc. HCI auf pH 2 gestellt.

Man extrahiert mit Methyl-tert.butylether (1x 2 L; 2x 1 L); die Phasen trennen sich weitgehend sauber ab und können leicht trüb sein. Bei der 3. Extraktion sind in der Regel beide Phasen klar.

Die vereinigten MTB-Extrakte werden zum Entwässern 2x mit je 250 ml gesättigter NaCl-Lösung geschüttelt. Die organische Phase wird über Na₂SO₄ getrocknet und gewichtskonstant eingeengt. Auswaage: ca.70g HPLC:ca 99.5 Flä-%.

### Beispiel 4

### Herstellung des Na-Salzes

278.3 g (0.327 Mol) HPLC-gereinigtes Produkt aus Beispiel 3 werden in 4.8 L Methanol bei Raumtemperatur gelöst; die Lösung wird mit 760ml Wasser versetzt (sie wird milchig). Man stellt mit 1n NaOH (ca.300 ml) gegen eine geeichte Elektrode pH 5.8 ein und saugt die trübe Lösung über eine Seitzfilterplatte ab. Das klare Filtrat wird am Rotationsverdampfer i. Vak. eingeengt. Der Eindampfrückstand wird mit Toluol viermal nachdestilliert. Der trockne Eindampfrückstand wird mit 5.8 L Aceton übergossen und ca.1 Std. bei Raumtemperatur gerührt. Man saugt ab, wäscht mit wenig Aceton nach und trocknet bei ca. 50 °C.

Ausbeute: 278 g (97% d.Th.bez auf die gereinigte freie Säure) HPLC:99.45 Flä-%.

### Beispiel 5

### Herstellung von Na-Salz durch Fällen aus Toluollösung

3 g (0.0978 Mol) gereinigte freie Säure aus Beispiel 3 werden in 500 ml Toluol gelöst. Eine leicht trübe Lösung wird über ein Seitzfilter filtriert. Die Toluollösung wird mit der zur Salzbildung erforderlichen Menge (die zuvor an einem aliquoten Teil ermittelt wird) 30 proz. Natriummethylatlösung unter Rühren versetzt. Die klare, leicht gelbliche Lösung wird langsam unter Rühren in 3 L Aceton getropft. Man rührt nach, gegebenfalls über Nacht, bis der Niederschlag pulvrig ist. Der Niederschlag wird abgesaugt; gegen Ende des Saugens kann die Nutsche verkleben. Man spült mit etwas Aceton nach und trocknet das leicht klebrige Produkt bei 50 °C.
Ausbeute 66,2 g (88,1% d.Th.)

### Beispiel 6

### Herstellung von (3'-Desoxv-3'-azido-thvmidin)-5'-phosphorsäure-(3-dodecylthio-2-decyloxy)-propylester unter Verwendung von Benzolsulfochlorid

17.3 g (0.034 Mol) 3-Dodecylthio-2-decyloxy-propanol-1-monophosphat werden analog Beispiel 1 in 100 ml abs. Pyridin gelöst, mit 6.5 ml (0.051 Mol) Benzolsulfochlorid versetzt und 4 Std. bei Raumtemperatur nachgerührt. Man setzt dann 8 g (0.03 Mol) AZT zu und verfährt weiter analog Beispiel 1. Das Rohprodukt (24.5 g;"109%" d.Th. bez.auf AZT) wird analog Beispiel 3 bis 5 in das Na-Salz überführt. Ausbeute 12.2 g (52.9% d. Th. bez auf AZT) HPLC: 99.43 Fl-%.

### Beispiel 7

### Herstellung des Ca-Salzes von (3'-Desoxy-3'-azido-thymidin)-5'-phosphorsäure-(3-dodecylthio-2-decyloxy)propylester unter Verwendung von 2,4,6-Trimethylbenzolsulfochlorid

86.8 g (0.17 Mol) 3-Dodecylthio-2-decyloxy-propanol-1-monophosphat werden analog Beispiel 6 in 515ml abs. Pyridin mit 57.7 g (0.26 Mol) 2.4.6-Trimethylbenzolsulfochlorid reagieren gelassen und dann nach Beispiel 1 mit 41.2 g (0.154 Mol) AZT umgesetzt. Das analog Beispiel 1 erhaltene Rohprodukt (138 g; "119" % d.Th. bez. auf AZT) wird analog Beispiel 2 in das rohe Ca-Salz überführt. Ausbeute 98.2 g (83.2% d.Th. bez.auf AZT)

### Beispiel 8

### Herstellung von (3'-Desoxy-3'-fluor-thymidin)-5'-phosphorsäure-(3-dodecylthio-2-decyloxy)propylester-Natriumsalz

Analog zu Beispiel 1 werden 78.9 g (0.159 Mol) 3-Dodecylthio-2-decyloxypropanol-1-monophosphat in 480ml abs. Pyridin in Gegenwart von 72.55 g (0.239 Mol) 2,4,6-Trisopropylbenzolsulfochlorid mit 35 g (0.143 Mol) 3'-Desoxy-3'-fluor-thymidin (FLT) umgesetzt. Auswaage 136 g Rohprodukt (130% bez. auf FLT). Das Rohprodukt wird analog Beispiel 2 als Calciumsalz gefällt, der Niederschlag in die freie Säure überführt, durch präparative Säulenchromatographie an RP-18 analog Beispiel 3 gereinigt und analog Beispiel 4 + 5 in das Natriumsalz überführt. Ausbeute 74 g (69% d. Th. bezogen auf FLT), HPLC: 99.41 Fl.-%.

### Beispiel 9

### Herstellung von (2'.3'-Didesoxy-inosin)-5'-phosphorsäure-(3-dodecylthio-2-decyloxy)propylester-Natriumsalz

Analog zu Beispiel 6 werden 15.8 g (32 mMol) 3-Dodecylthio-2-decyloxypropanol-1-monophosphat in 100 ml abs. Pyridin mit 14.5g (48 mMol) 2,4,6-Triisopropylbenzolsulfochlorid versetzt und nach 4 Std. mit 6.77 g (29 mMol) 2',3'-Didesoxy-inosin (DDI) zur Reaktion gebracht. Das Rohprodukt wird analog Beispiel 3 bis 5 in das Natriumsalz überführt. Ausbeute 13.04 g (61% d. Th. bezogen auf DDI), HPLC: 99.4 Fl.-%.

### Beispiel 10

### Herstellung von (3'-Desoxy-3'-azido-thymidin)-5'-phosphorsäure-[2,3-bis-(undecyloxy)]propylester-Natriumsalz

Analog zu Beispiel 1 werden 15.8 g (32 mMol) 2,3 Bis-(undecyloxy)propanol-1-monophosphat in 96ml abs. Pyridin gelöst und in Gegenwart von 14.5 g (48 mMol) 2,4,6-Triisopropylbenzolsulfochlorid mit 6.77 g (29 mMol) 3'-Azido-3'-desoxy-thymidin (AZT) zur Reaktion gebracht. Das Rohprodukt wird analog Beispiel 3 bis 5 in das Natriumsalz überführt. Ausbeute 17.15 g (77% d. Th. bezogen auf AZT), HPLC: 99.7 FI.-%.

### Beispiel 11

Folgende Verbindungen wurden analog zu Bsp. 1 als Rohprodukt hergestellt, analog Bsp. 2 über das Calciumsalz angereichert, analog Bsp. 3 durch präp. HPLC gereinigt und analog Bsp. 5 in das Natriumsalz überführt:
1. (3'-Desoxy-3'-azidothymidin)-5'-phosphorsäure-(3-undecylmercapto-2-undecyloxy)propylester-Natriumsalz
   Schmp. 218-222 °C Zers., R_{f}= 0.55 (Laufmittel: Isopropanol/Butylacetat/Wasser = 5/3/2)
2. (3'-Desoxy-3'-azidothymidin)-5'-phosphorsäure-(3-dodecyloxy-2-decyloxy)-propylester-Natriumsalz
   Schmp. 205-211 °C Zers., R_{f}= 0.60 (Laufmittel: Isopropanol/Butylacetat/Wasser = 5/3/2)
3. (3'-Desoxy-3'-azidothymidin)-5'-phosphorsäure-(3-decylmercapto-2-dodecyloxy)propylester-Natriumsalz
   Schmp. >206 °C Zers., R_{f}= 0.24 (Laufmittel: MeOH/H₂O = 8/2)
4. (3'-Desoxy-3'-azidothymidin)-5'-phosphorsäure-(3-decylmercapto-2-decyloxy)propylester-Natriumsalz
   Schmp. >207 °C Zers., R_{f}= 0.45 (Laufmittel: Isopropanol/Butylacetat/Wasser = 5/3/2)
5. (3'-Desoxy-3'-azidothymidin)-5'-phosphorsäure-hexadecyl-ester-Natriumsalz
   Schmp. 227-230 °C Zers., R_{f}= 0.55 (Laufmittel: Isopropanol/Butylacetat/Wasser = 5/3/2)
6. (3'-Desoxy-3'-azidothymidin)-5'-phosphorsäure-(3-tetradecylmercapto-2-decyloxy)propylester-Natriumsalz
   Schmp. 155 °C, R_{f}= 0.30 (Laufmittel: Essigester/Methanol = 3/1)
7. (3'-Desoxy-3'-azidothymidin)-5'-phosphorsäure-(3-dodecylmercapto)-propylester-Natriumsalz
   Schmp. >200 °C Zers., R_{f}= 0.20 (Laufmittel: Essigester/Methanol = 3/1)
8. (3'-Desoxy-3'-azidothymidin)-5'-phosphorsäure-(3-dodecylmercapto-2-dodecyloxy)propylester-Natriumsalz
   Schmp. >170 °C Zers., R_{f}= 0.25 (Laufmittel: Essigester/Methanol = 3/1)
9. (3'-Desoxy-3'-azidothymidin)-5'-phosphorsäure-(3-decylmercapto-2-hexadecyloxy)propylester-Natriumsalz
   Schmp. 135 °C, R_{f}= 0.35 (Laufmittel: Essigester/Methanol = 3/1)
10. (3'-Desoxy-3'-azidothymidin )-5'-phosphorsäure-(2-dodecyloxy)tetradecylester-Natriumsalz
   Schmp. 83 °C, R_{f}= 0.35 (Laufmittel: Essigester/Methanol = 3/1)
11. (3'-Desoxy-3'-azidothymidin)-5'-phosphorsäure-(3-tridecylmercapto-2-decyloxy)propylester-Natriumsalz
   Schmp. >190 °C Zers., R_{f}= 0.20 (Laufmittel: Essigester/Methanol = 3/1)
12. (3'-Desoxy-3'-azidothymidin)-5'-phosphorsäure-(3-dodecylmercapto-2-octyloxy)propylester-Natriumsalz
   Schmp. >200 °C Zers., R_{f}= 0.60 (Laufmittel: Methylenchlorid/Methanol/Wasser = 65/25/4)*
13. (3'-Desoxy-3'-azidothymidin )-5'-phosphorsäure-( 3-undecylmercapto-2-decyloxy)propylester-Natriumsalz
   Schmp. >200 °C Zers., R_{f}= 0.25 (Laufmittel: Essigester/Methanol = 3/1)
14. (3'-Desoxy-thymidin)-5'-phosphorsäure-(3-dodecylmercapto-2-decyloxy)-propylester-Natriumsalz
   Schmp. >175 °C Zers., R_{f}= 0.45 (Laufmittel: Isopropanol/Butylacetat/Wasser= 5/3/2)
15. Thymidin-5'-phosphorsäure-(3-dodecylmercapto-2-decyloxy)propylester-Natriumsalz
   Schmp. 251-254 °C Zers., R_{f}= 0.45 (Laufmittel: n-Propanol/H₂O = 9/1)
16. (6-Mercaptopurin-9-β-D-ribofuranosid)-5'-phosphorsäure-(3-dodecylmercapto-2-decyloxy)propylester-Natriumsalz
   Schmp. >200 °C Zers., R_{f}= 0.45 (Laufmittel: Isopropanol/Butylacetat/konz. Ammoniak/Wasser = 50/30/5/15)
17. (5-Fluoruridin)-5'-phosphorsäure-(3-dodecylmercapto-2-decyloxy)propylester-Natriumsalz
   Schmp. >210 °C Zers., R_{f}= 0.55 (Laufmittel: Methylenchlorid/Methanol/Wasser = 65/25/4)
18. (2',3'-Didesoxy-cytidin)-5'-phosphorsäure-(3-dodecylmercapto-2-decyloxy)- propylester-Natriumsalz
   Schmp. 202-205 °C Zers., R_{f}= 0.50 (Laufmittel: Isopropanol/Butylacetat/Wasser = 5/3/2)
19. (3'-Desoxy-3'-fluorthymidin)-5'-phosphorsäure-(3-undecylmercapto-2-undecyloxy)propylester-Natriumsalz
   Schmp. >200 °C Zers., R_{f}= 0.15 (Laufmittel: CH₂Cl₂/MeOH = 8/2)
20. (6-Methylmercaptopurin-9-β-D-ribofuranosid)-5'-phosphorsäure-(3-dodecylmercapto-2-decyloxy)-propylester-Natriumsalz
   Schmp. > 170 °C Zers., R_{f} = 0.22 (Laufmittel: Isopropanol/Butylacetat/Wasser/konz. Ammoniak 50/30/15/5)
21 . 2'-(9-{[(1 -Hydroxymethyl )ethoxy]methyl}guanin)-phosphorsäure-(3-dodecylmercapto-2-decyloxy)-propylester-Natriumsalz
   R_{f} = 0.73 (Laufmittel: H₂O/MeOH 0.5/9.5 auf RP-8),
   R_{f} = 0.30 (Laufmittel: CH₂-Cl₂/MeOH/H₂O 6.5/2.5/0.4 auf Kieselgel)
22. 2'-[9-(Ethoxymethyl)guanin]-phosphorsäure-(3-dodecylmercapto-2-decyloxy)propylester-Natriumsalz
   R_{f} = 0.77 (Laufmittel: H₂O/MeOH 0.5/9.5 auf RP-8),
   R_{f} = 0.35 (CH₂-Cl₂/MeOH/H₂O 6.5/2.5/0.4 auf Kieselgel)

## Patentansprüche

1. Verfahren zur Herstellung von salzen unsymmetrischer Phosphorsäurediestern, dadurch gekennzeichnet, daß man
[a] einen Phosphorsäureester der Formel I,
R¹-O-P(O)(OH)₂ (I),
in dem R¹ einen Lipidrest der allgemeinen Formel IV bedeutet 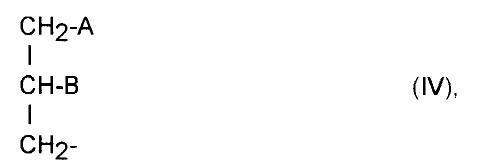
in der A und B gleich oder verschieden sein können und Wasserstoff, C₁-C₁₈-Alkyl, C₁-C₁₈-Alkoxy, C₁-C₁₈-Alkylthio, C₁-C₁₈-Alkylsulfinyl oder C₁-C₁₈-Alkylsulfonyl bedeuten,
oder in dem R¹ eine geradkettige oder verzweigte, gesättigte oder ungesättigte Alkylkette mit 10-20 Kohlenstoffatomen bedeutet, die gegebenenfalls ein- oder mehrfach substituiert sein kann durch Halogen, C₁-C₆-Alkoxy, C₁-C₆-Alkylmercapto
mit einer Verbindung II
R² - OH (II)
in der R² einen organischen Rest darstellt.
in Gegenwart eines Arylsulfonsäurechlorids, eines sterisch gehinderten Carbonsäurechlorids oder Phosphorsäurechlorids und einer organischen Base kondensiert,
[b] den entstandenen Phosphorsäurediester durch Zugabe einer Erdalkali-lonen-haltigen Lösung als schwerlösliches Salz fällt und isoliert,
[c] das schwerlösliche Salz durch Suspendieren in einem mit Wasser nicht mischbaren organischen Lösemittel und einer verdünnten wäßrigen Mineralsäure als freie Säure im organischen Lösemittel isoliert,
[d] das nach den Schritten [a] - [c] erhaltene Rohprodukt durch präparative Chromatographie reinigt,
[e] anschließend die als freie Säure erhaltene Verbindung in ein beliebiges physiologisch verträgliches Salz überführt.

2. Verfahren gemäß Anspruch 1, dadurch gekennzeichnet, daß die Erdalkalilonen-haltige Lösung eine Calciumionen-haltige Lösung ist.

3. Verfahren gemäß einem der Ansprüche 1 oder 2, dadurch gekennzeichnet, daß als Arylsulfonsäurechlorid Benzol-, Toluol-, 2,4,6-Trimethylbenzol-, 2,6-Dimethylbenzol-, 2,4,6-Triisopropylbenzol- oder 2,6-Diisopropylbenzol-sulfonsäurechlorid eingesetzt wird.

4. Verfahren gemäß einem der Ansprüche 1-3, dadurch gekennzeichnet, daß ein Phosphorsäureester eingesetzt wird, in dem R¹ einen Lipidrest der allgemeinen Formel IV bedeutet, in dem A und B verschieden voneinander sind und C₁₀-C₁₆-Alkoxy und C₁₀-C₁₆-Alkylthio bedeuten.

5. Verfahren gemäß einem der Ansprüche 1-4, dadurch gekennzeichnet, daß eine Verbindung II eingesetzt wird, in der R² einen 5'-Nucleosidrest der allgemeinen Formel V darstellt 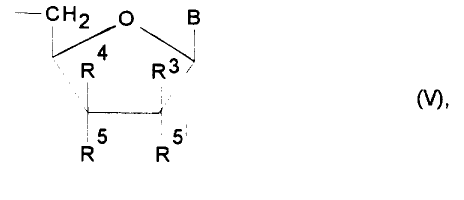 in der
R³ Wasserstoff, Halogen oder eine Hydroxygruppe,
R⁴, R⁵ jeweils Wasserstoff oder einer der Reste R⁴ und R⁵ Halogen, eine Hydroxy-, eine Cyano-, eine Amino- oder eine Azidogruppe bedeuten und außerdem R³ und R⁴ eine weitere Bindung zwischen C-2' und C-3' darstellen können,
_{R}5' Wasserstoff, Hydroxy, Azido, Amino, Cyano oder Halogen bedeutet,
oder einen seco-Nucleosidrest der allgemeinen Formel V a darstellt 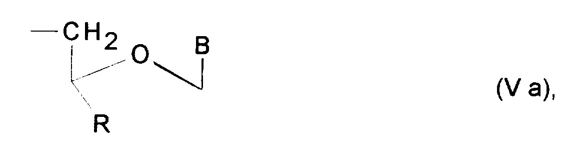
in dem
R ein Wasserstoffatom oder eine gegebenenfalls durch Hydroxy, Halogen oder Azido substituierte C₁-C₃-Alkylgruppe darstellt,
und
B eine der folgenden Verbindungen bedeutet:
1.) 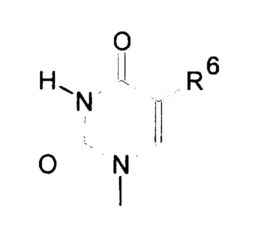 wobei
R⁶ Wasserstoff, eine Alkylkette mit 1 - 4 Kohlenstoffatomen, die durch Halogen substituiert sein kann, ein Alkenyl- bzw. Alkinylrest mit 2 - 6 Kohlenstoffatomen, der gegebenenfalls durch Halogen substituiert sein kann, oder Halogen sein kann,
2.) 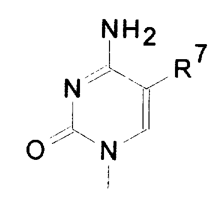 wobei
R⁷ Wasserstoff, eine Alkylkette mit 1 - 4 Kohlenstoffatomen, die durch Halogen substituiert sein kann, oder Halogen sein kann,
3.) 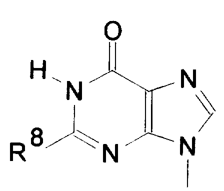 wobei
R⁸ Wasserstoff, eine Alkylkette mit 1 - 4 Kohlenstoffatomen, Halogen, oder eine Hydroxy- oder eine Aminogruppe sein kann,
4.) 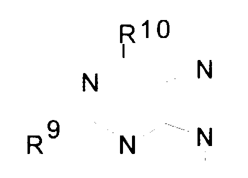 wobei
R⁹ Wasserstoff, Halogen oder eine Aminogruppe sein kann, und
R¹⁰ Wasserstoff, Halogen, Mercapto, C₁-C₆-Alkoxy, C₁-C₆-Alkylmercapto, oder eine Aminogruppe, die mono- oder disubstituiert sein kann durch C₁-C₆-Alkyl-, C₁-C₆-Alkoxy-, Hydroxy-C₂-C₆-alkyl- und/oder C₃-C₆-Cycloalkyl-, Aryl-, Hetaryl-, Aralkyl- oder Hetarylalkylgruppen, die gegebenenfalls im Aryl- oder Hetarylrest noch durch eine oder mehrere Hydroxy-, Methoxy- oder Alkylgruppen oder Halogen substituiert sein können, oder Allyl, das gegebenenfalls mit Mono- oder Dialkyl- oder Alkoxygruppen substituiert sein kann, bedeutet.

6. Verfahren gemäß Anspruch 5, dadurch gekennzeichnet, daß eine Verbindung II eingesetzt wird, in der R² den 3'-Desoxy-3'-azidothymidin-, 3'-Desoxy-3'-fluorthymidin-, 5-Fluoruridin-, 6-Mercaptopurin-9-β-D-ribofuranosid-, 6-Methylmercaptopurin-9-β-D-ribofuranosid-, 9-{[(1-Hydroxymethyl)ethoxy]methyl}guanin- oder 9-(Ethoxymethyl)guanin-Rest darstellt.

## Claims

1. Process for the production of salts of asymmetrical phosphoric acid diesters, wherein
[a] a phosphoric acid ester of formula I,
R¹-O-P(O)(OH)₂ (I)
in which R¹ denotes a lipid residue of the general formula IV 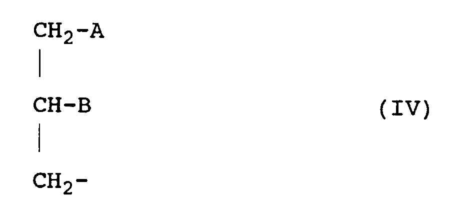
in which A and B can be the same or different and denote hydrogen, C₁-C₁₈ alkyl, C₁-C₁₈ alkoxy, C₁-C₁₈ alkylthio, C₁-C₁₈ alkylsulfinyl or C₁-C₁₈ alkylsulfonyl
or in which R¹ denotes a straight-chained or branched, saturated or unsaturated alkyl chain with 10-20 carbon atoms which can be optionally substituted once or several times by halogen C₁-C₆ alkoxy, C₁-C₆ alkylmercapto
is condensed with a compound II
R² - OH (II)
in which R² represents an organic residue,
in the presence of an arylsulfonic acid chloride or a sterically hindered acyl chloride or phosphoric acid chloride and an organic base,
[b] the phosphoric acid diester which is formed is precipitated as a sparingly soluble salt by the addition of a solution containing alkaline-earth ions and it is isolated,
[c] the sparingly soluble salt is isolated as the free acid in an organic solvent by suspension in a water-immiscible organic solvent and a dilute aqueous mineral acid,
[d] the crude product obtained after steps [a] - [c] is purified by preparative chromatography,
[e] subsequently the compound obtained as a free acid is converted into any desired physiologically tolerated salt.

2. Process as claimed in claim 1, wherein the solution containing alkaline-earth ions is a solution containing calcium ions.

3. Process as claimed in one of the claims 1 or 2, wherein benzene-, toluene-, 2,4,6-trimethylbenzene-, 2,6-dimethylbenzene-, 2,4,6-triisopropylbenzene- or 2,6-diisopropylbenzene-sulfonic acid chloride is used as the arylsulfonic acid chloride.

4. Process as claimed in one of the claims 1 - 3, wherein a phosphoric acid ester is used in which R¹ denotes a lipid residue of the general formula IV in which A and B are different from one another and denote C₁₀-C₁₆ alkoxy and C₁₀-C₁₆ alkylthio.

5. Process as claimed in one of the claims 1 - 4, wherein a compound II is used in which R² represents a 5'-nucleoside residue of the general formula V 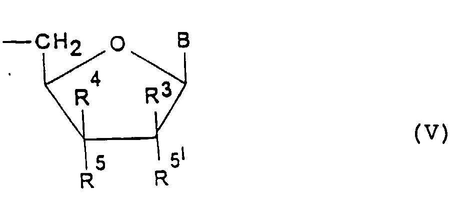 in which
R³ denotes hydrogen, halogen or a hydroxy group,
R⁴, R⁵ each denote hydrogen or one of the residues R⁴ and R⁵ denotes halogen, a hydroxy, a cyano, an amino or an azido group and R³ and R⁴ in addition can represent a further bond between C-2' and C-3',
R^{5'} denotes hydrogen, hydroxy, azido, amino, cyano or halogen
or it represents a seco-nucleoside residue of the general formula Va 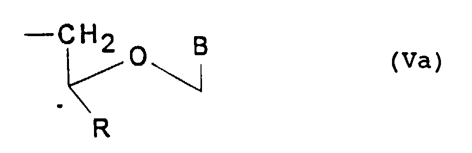
in which
R represents a hydrogen atom or a C₁-C₃ alkyl group which is optionally substituted by hydroxy, halogen or azido
and
B denotes one of the following compounds:
1.) 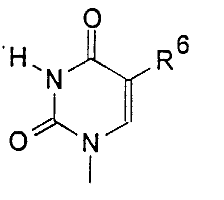 in which
R⁶ can be hydrogen, an alkyl chain with 1-4 carbon atoms which can be substituted by halogen, an alkenyl or alkinyl residue with 2 - 6 carbon atoms which can be optionally substituted by halogen, or it can be halogen,
2.) 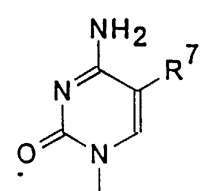 in which
R⁷ can be hydrogen, an alkyl chain with 1 - 4 carbon atoms which can be substituted by halogen or it can be halogen
3.) 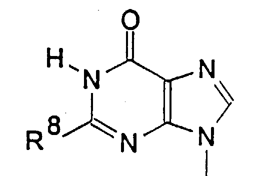 in which
R⁸ can be hydrogen, an alkyl chain with 1 - 4 carbon atoms, halogen or a hydroxy or amino group
4.) 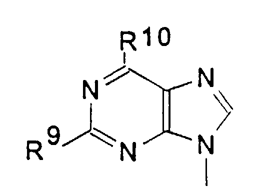 in which
R⁹ can be hydrogen, halogen or an amino group and
R¹⁰ denotes hydrogen, halogen, mercapto, C₁-C₆ alkoxy, C₁-C₆ alkylmercapto or an amino group which can be mono- or disubstituted by C₁-C₆ alkyl, C₁-C₆ alkoxy, hydroxy-C₂-C₆ alkyl and/or C₃-C₆ cycloalkyl, aryl, hetaryl, aralkyl or hetarylalkyl groups which can be further substituted if desired in the aryl or hetaryl residue by one or several hydroxy, methoxy or alkyl groups or by halogen or it denotes allyl which can be substituted if desired by monoalkyl or dialkyl or alkoxy groups.

6. Process as claimed in claim 5, wherein a compound II is used in which R² represents a 3'-deoxy-3'-azidothymidine, 3'-deoxy-3'-fluorothymidine, 5-fluorouridine, 6-mercaptopurine-9-β-D-ribofuranoside, 6-methylmercaptopurine-9-β-D-ribofuranoside, 9-{[(1-hydroxymethyl)ethoxy]methyl}guanine or 9-(ethoxymethyl)guanine residue.

## Revendications

1. Procédé de préparation de sels de diesters asymétriques d'acide phosphorique, caractérisé par le fait que
[a] un ester d'acide phosphorique de formule I
R¹-O-P(O)(OH)₂ (I),
dans laquelle R¹ représente un reste lipide de formule générale IV 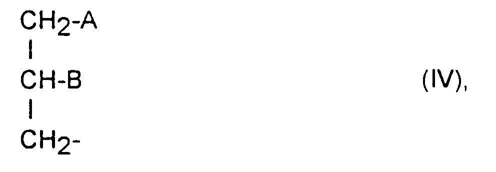
où A et B peuvent être identiques ou différents et représentent un atome d'hydrogène, un groupe alkyle en C₁-C₁₈, alcoxy en C₁-C₁₈, alkylthio en C₁-C₁₈, alkylsulfinyle en C₁-C₁₈ ou alkylsulfonyle en C₁-C₁₈,
ou dans laquelle R¹ représente une chaîne alkylique saturée ou insaturée, linéaire ou ramifiée, ayant 10-20 atomes de carbone, qui peut être éventuellement substituée une ou plusieurs fois avec un atome d'halogène, un groupe alcoxy en C₁-C₆, alkylmercapto en C₁-C₆,
est condensé avec un composé II
R²-OH (II),
où R² représente un résidu organique,
en présence d'un chlorure d'acide arylsulfonique ou d'un chlorure d'acide carboxylique ou phosphorique stériquement encombré et d'une base organique,
[b] le phosphodiester formé précipite, par addition d'une solution contenant des ions alcalino-terreux, sous forme de sel peu soluble et il est isolé sous cette forme,
[c] le sel peu soluble, mis en suspension dans un solvant organique non miscible à l'eau et un acide minéral en solution aqueuse diluée, est isolé sous forme d'acide libre dans le solvant organique,
[d] le produit brut obtenu dans les étapes [a] - [c] est purifié par chromatographie préparative,
[e] ensuite, le composé obtenu sous forme d'acide libre est transformé en un sel quelconque physiologiquement acceptable.

2. Procédé selon la revendication 1, caractérisé par le fait que la solution contenant les ions alcalino-terreux est une solution contenant des ions calcium.

3. Procédé selon l'une quelconque des revendications 1 ou 2, caractérisé par le fait qu'en tant que chlorure d'acide arylsulfonique, on utilise le chlorure d'acide benzènesulfonique, toluènesulfonique, 2,4,6-triméthylbenzènesulfonique, 2,6-diméthylbenzènesulfonique, 2,4,6-triisopropylbenzènesulfonique ou 2,6-diisopropylbenzènesulfonique.

4. Procédé selon l'une quelconque des revendications 1-3, caractérisé par le fait que l'on utilise un ester d'acide phosphorique dans lequel R¹ représente un reste lipide de formule générale IV où A et B sont différents l'un de l'autre et représentent un groupe alcoxy en C₁₀-C₁₆ et alkylthio en C₁₀-C₁₆.

5. Procédé selon l'une quelconque des revendications 1-4, caractérisé par le fait que l'on utilise un composé II dans lequel R² représente un résidu de 5'-nucléoside de formule générale V 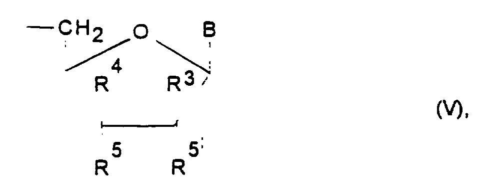 dans laquelle
R³ représente un atome d'hydrogène. d'halogène ou un groupe hydroxy.
R⁴, R⁵ représentent chacun un atome d'hydrogène, ou l'un des restes R⁴ et R⁵ représente un atome d'halogène, un groupe hydroxy, cyano, amino ou azido, et en outre, R³ et R⁴ peuvent représenter une liaison supplémentaire entre C-2' et C-3',
R^{5'} représente un atome d'hydrogène, un groupe hydroxy, azido, amino, cyano ou halogéno,
ou un résidu seco-nucléoside de formule générale Va 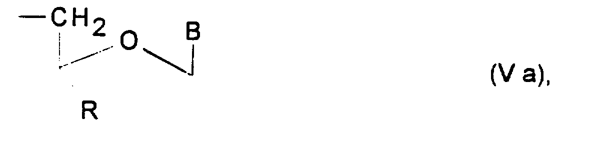
dans laquelle
R représente un atome d'hydrogène ou un groupe alkyle en C₁-C₃ éventuellement substitué par un groupe hydroxy, halogéno ou azido,
et
B représente un des composés suivants :
1.) 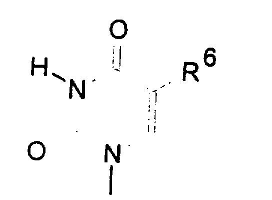 où
R⁶ représente un atome d'hydrogène, une chaîne alkyle ayant 1-4 atomes de carbone, qui peut être substituée par un atome d'halogène, un reste alcényle ou alcinyle ayant 2-6 atomes de carbone qui peut être éventuellement substitué par un atome d'halogène, ou un atome d'halogène,
2.) 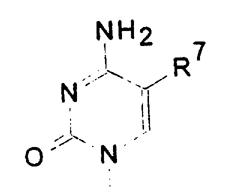 où
R⁷ représente un atome d'hydrogène, une chaîne alkyle ayant 1-4 atomes de carbone qui peut être substituée par un atome d'halogène, ou un atome d'halogène,
3.) 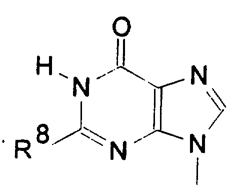 où
R⁸ représente un atome d'hydrogène, une chaîne alkyle ayant 1-4 atomes de carbone, un atome d'halogène, ou qui peut être un groupe hydroxy ou amino,
4.) 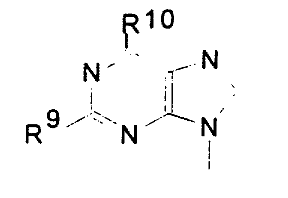 où
R⁹ peut être un atome d'hydrogène, d'halogène ou un groupe amino, et
R¹⁰ peut être un atome d'hydrogène. d'halogène, un groupe mercapto, alcoxy en C₁-C₆, alkylmercapto en C₁-C₆, ou un groupe amino, qui peut être mono- ou disubstitué par un groupe alkyle en C₁-C₆, alcoxy en C₁-C₆, hydroxy, alkyle en C₂-C₆ et/ou cycloalkyle en C₃-C₆, aryle, hétéroaryle, aralkyle ou hétéroaralkyle, qui peuvent être encore substitués éventuellement sur le reste aryle ou hétéroaryle par un ou plusieurs groupes hydroxy, méthoxy ou alkyle, ou par un atome d'halogène, ou allyle, qui peut être éventuellement substitué par des groupes mono- ou dialkyle ou alcoxy.

6. Procédé selon la revendication 5, caractérisé par le fait que l'on utilise un composé II dans lequel R² représente un résidu 3'-désoxy-3'-azidothymidine, 3'-désoxy-3'-fluorothymidine, 5-fluoruridine, 6-mercaptopurine-9-β-D-ribofuranoside, 6-méthylmercaptopurine-9-β-D-ribofuranoside, 9-{[(1-hydroxyméthyl)éthoxy]méthyl}-guanine ou 9-(éthoxyméthyl)guanine.
